# FASCICULE DE BREVET EUROPEEN

(11) **EP 2 135 631 B1**
(45) Date de publication et mention de la délivrance du brevet: **26.09.2012**
(21) Numéro de dépôt: 09162177.1
(22) Date de dépôt: 08.06.2009
(51) Int. Cl.: A61M 5/32

(54) **Dispositif de sécurité pour un dispositif d'injection**
Sicherheitsvorrictung für eine Injektionsvorrichtung
Security device for an injection device

(30) Priorité: 06.06.2008 FR 0853767
(43) Date de publication de la demande: 23.12.2009
(73) Titulaire: Rexam Pharma La Verpillière, 38292 La Verpillière Cedex (FR)
(72) Inventeur: Painchaud, Gaetan, 69340, FRANCHEVILLE (FR); Dugand, Pascal, 38780, ESTRABLIN (FR); Xavier, Julia, 38090, VILLEFONTAINE (FR)
(74) Mandataire: Thiollier, Clémence-Olivia Laure Marie

(56) Documents cités:
- WO-A-2007/138299
- DE-A1- 10 359 694
- FR-A- 2 778 853
- US-A1- 2005 159 706

## Description

La présente invention concerne le domaine des dispositifs de sécurité pour des dispositifs d'injection. Plus précisément mais non exclusivement, ces dispositifs de sécurité sont intégrés dans des dispositifs d'injection d'un produit dans le corps d'un sujet, ce produit pouvant prendre la forme d'un liquide, contenu dans une seringue d'injection, ou encore la forme d'un implant, c'est-à-dire d'un composé à l'état solide ou semi-solide, injecté de façon intra-musculaire ou sous-cutanée.

On connaît déjà un dispositif de sécurité pour dispositif d'injection, tel que ceux décrits dans les documents FR 2 830 765 et FR 2 778 853, muni d'une seringue ou d'un réservoir portant une aiguille et d'un fourreau protecteur de l'aiguille. Ce fourreau peut prendre une position d'injection, laissant découverte l'aiguille, ou une position de sécurité, dans laquelle il recouvre l'aiguille. Le fourreau est entraîné par un ressort, comprimé lorsque le fourreau est en position d'injection, et détendu lorsque le fourreau est en position de sécurité. Afin de contrôler le mouvement du ressort, le dispositif de sécurité est muni de pattes escamotables retenant le ressort en position comprimée au cours de l'injection.

Avant son utilisation, le dispositif de sécurité est généralement stocké en position d'injection, donc dans une position dans laquelle le ressort est mis sous contrainte.

On constate que les pattes escamotables retenant le ressort peuvent être endommagées par usure au cours du stockage du dispositif, d'autant plus que ce stockage peut s'étaler sur plusieurs années. Plus précisément, les pattes escamotables sont souvent réalisées en matière plastique souple et peuvent subir un certain fluage au cours du temps, d'autant plus qu'elles subissent une tension de la part du ressort comprimé qui exerce une certaine contrainte dessus. Il en ressort que le dispositif de sécurité peut être défaillant s'il n'est pas utilisé au bout d'un certain temps.

L'invention a notamment pour but de résoudre cet inconvénient en proposant un dispositif de sécurité dont le bon fonctionnement est garanti même après un stockage relativement long avant utilisation.

A cet effet, l'invention a pour objet un dispositif de sécurité pour dispositif d'injection tel que défini dans la revendication 1.

Ainsi, on propose d'épargner les moyens d'escamotage au cours du stockage du dispositif de sécurité, en leur évitant que ces moyens de rappel soient mis sous tension, c'est-à-dire en leur évitant de subir la contrainte exercée par les moyens de rappel sur les moyens d'escamotage lorsque le dispositif de sécurité est en configuration d'injection.

Les moyens de désactivation des moyens escamotables, dans la configuration de stockage, exercent sur les moyens de rappel une contrainte supérieure à la contrainte exercée par les moyens escamotables en configuration d'injection, de façon que les moyens escamotables ne soient pas sollicités lors du stockage.

Par exemple, les moyens de rappel sont un ressort en compression, prenant une position comprimée, donc sous contrainte, dans la configuration d'injection du dispositif, et une position détendue dans la configuration de sécurité, de façon à pousser le fourreau pour recouvrir l'aiguille. Selon cet exemple, les moyens de désactivation des moyens escamotables exercent sur le ressort une compression supérieure à la compression exercée en configuration d'injection. Ainsi, le ressort étant davantage comprimé en configuration de stockage, il ne "tire" plus sur les moyens escamotables.

En évitant de mettre les moyens escamotables sous tension lors de leur stockage, on diminue considérablement les risques de fluage de leur matière, donc les risque de défaillance du dispositif de sécurité.

On comprendra que les moyens de désactivation des moyens escamotables agissent avantageusement de façon que les moyens escamotables désactivés, donc en configuration de stockage, n'exercent plus leur fonction de retenue des moyens de rappel. On notera par ailleurs que les trois configurations précitées du dispositif de sécurité (configurations d'injection, de sécurité et de stockage) correspondent à des configuration distinctes les unes des autres.

Parmi les autres avantages du dispositif de sécurité, on notera que, du fait qu'on épargne les moyens escamotables, il n'est pas nécessaire que ces moyens escamotables soient aussi robustes que lorsqu'ils sont mis sous contrainte lors de leur stockage, si bien que l'on peut prévoir des moyens escamotables de forme différente, notamment de dimension et d'épaisseur réduites. En outre, on peut prévoir un matériau moins résistant au fluage que les matériaux utilisés jusqu'à présent, en particulier un matériau moins cher. Par ailleurs, par ce stockage au cours duquel les moyens escamotables sont désactivés, on diminue les risques que le dispositif de sécurité se déclenche accidentellement au cours du stockage. Ceci est particulièrement avantageux d'une part au cours du transport, car les vibrations ou les chocs peuvent déclencher accidentellement le dispositif, d'autre part au cours des opérations de stérilisation, lesquelles peuvent faire subir aux moyens escamotables un fluage qui peut altérer leur fonctionnement, voire les escamoter accidentellement. Enfin, le dispositif de sécurité proposé évite d'avoir à vérifier le non-endommagement du dispositif avant son utilisation.

Le dispositif de sécurité peut en outre comporter l'une ou plusieurs des caractéristiques suivantes.
- Les moyens escamotables de retenue des moyens de rappel comprennent des ergots escamotables, ces ergots étant en butée contre une surface lorsque le dispositif est en configuration d'injection, et escamotés de cette butée lorsque dispositif est en configuration de sécurité ; les moyens de désactivation maintenant les ergots à une certaine distance de la butée lorsque le dispositif est en configuration de stockage. Ainsi, la désactivation des moyens escamotables revient à les désengager de la butée grâce à laquelle ils assurent la retenue des moyens de rappel.
- Les moyens de désactivation comprennent un capuchon de protection recouvrant l'aiguille lorsque le dispositif est en configuration de stockage, le montage du capuchon sur le dispositif de sécurité déclenchant la désactivation des moyens escamotables, de préférence en déplaçant le fourreau protecteur. Ainsi, le montage du capuchon sur le dispositif d'injection permet de lui donner automatiquement sa configuration de stockage. Puis, le démontage du capuchon, juste avant l'injection, stoppe automatiquement l'action des moyens de désactivation, si bien que les moyens escamotables sont activés et que le dispositif d'injection passe en configuration d'injection. Par exemple, le capuchon est vissé sur le dispositif d'injection et le fourreau porte les moyens escamotables ; le vissage du capuchon déplace légèrement le fourreau, donc les moyens escamotables, de préférence en éloignant les moyens escamotables de la butée avec laquelle ils sont destinés à coopérer. L'utilisation d'un tel capuchon est particulièrement astucieuse, du fait que l'on évite de prévoir une pièce supplémentaire portant les moyens de désactivation, le capuchon assurant à la fois la protection de l'aiguille en configuration de stockage et de désactivation des moyens escamotables.
- Les moyens de désactivation comprennent un organe de stockage monté sur l'extrémité proximale du dispositif de sécurité et appuyant, directement ou indirectement, sur les moyens de rappel pour leur faire prendre leur configuration de stockage. Ainsi, on rapporte une pièce sur l'extrémité proximale du dispositif, qui peut procurer plusieurs avantages. En effet, cette pièce peut assurer la protection ou le verrouillage du dispositif pendant le stockage, en empêchant ou en limitant le déplacement d'une tige de piston. En outre, cette pièce peut recouvrir l'extrémité proximale du dispositif, de façon à bloquer l'accès au produit contenu dans le dispositif. Par exemple, la pièce peut empêcher une personne malintentionnée de prélever du produit en faisant passer une aiguille de prélèvement à l'intérieur du piston depuis l'extrémité proximale.
- L'organe comprend des moyens amovibles de fixation sur le dispositif de sécurité et une partie distale, s'étendant depuis les moyens de fixationet formant entretoise entre les moyens de fixation et les moyens de rappel. Cette partie distale de l'organe peut former entretoise, soit par appui direct (avec contact) sur les moyens de rappel, soit par appui indirect, par exemple en appuyant sur la collerette d'une seringue sur laquelle agissent les moyens de rappel. Grâce à cette partie formant entretoise, on peut maintenir les moyens de rappel à une certaine distance des moyens escamotables, pour ne pas les solliciter pendant leur stockage.
- L'organe de stockage comprend des moyens de protection d'une tige de piston. Ces moyens de protection peuvent comprendre un logement de réception de la tige de piston, ouvert ou fermé, par exemple semi-tubulaire, configuré de façon à empêcher une courbure, une cassure ou encore un déplacement accidentels ou volontaires de la tige de piston au cours du stockage. En d'autres termes, le logement de réception forme un étui de protection de la tige de piston, limitant son accès. Ainsi, l'organe de stockage à une double fonction : d'une part épargner les moyens escamotables, d'autres part assurer la protection de la tige de piston.
- Le dispositif d'injection est un dispositif d'injection d'un implant à l'état solide ou semi-solide, de préférence un dispositif de rétro-injection de l'implant. Un dispositif de rétro-injection offre des spécificités techniques qui s'accompagnent d'une certaine complexité mécanique, si bien qu'il est particulièrement intéressant d'utiliser une solution simple telle que décrite ci-dessus pour garantir le bon fonctionnement du dispositif. En effet, un dispositif de rétro-injection de l'implant présente notamment des moyens d'injecter l'implant dans le corps du patient au cours du retrait de l'aiguille hors du corps du patient. La rétro-injection d'implant a pour avantage de ne pas exercer de contrainte sur l'implant au moment où ce dernier sort de l'aiguille pour rester dans le corps du patient, à la différence d'une injection simple où le produit est poussé, ou comprimé, dans la peau du patient. Au cours de la rétro-injection, l'implant remplit un vide laissé par l'aiguille au moment de son retrait. Ainsi le dispositif d'injection d'implant nécessite souvent des moyens particuliers pour assurer la retenue de l'implant avant l'injection et également pour maintenir l'implant en position dans la peau du patient au moment du retrait de l'aiguille.
- Le dispositif d'injection est un dispositif d'injection d'un liquide, comportant une seringue d'injection de liquide ou de gel. Eventuellement, la seringue est une seringue standardisée, en verre, ne comprenant aucun moyen spécifique pour assurer la sécurité, ces moyens étant portés par le dispositif de sécurité. Le dispositif de sécurité peut comprendre, outre le fourreau, une pièce intermédiaire dans laquelle la seringue est encliquetée, au moins lorsque le dispositif est en configuration de sécurité. De manière alternative, la seringue est en matière plastique, pas forcément de forme standard.

L'invention a également pour objet un dispositif d'injection comportant un dispositif de sécurité tel que décrit ci-dessus.

Eventuellement, comme mentionné précédemment, le dispositif d'injection est un dispositif d'injection d'un implant, et comporte des moyens de rétro-injection de l'implant, ou encore un dispositif d'injection d'un liquide, comportant une seringue d'injection de liquide.

L'invention a également pour objet un procédé d'assemblage de ce dispositif d'injection, le fourreau étant monté coulissant par rapport à un corps intermédiaire, et les moyens de rappel étant retenus, d'une part contre le fourreau, d'autre part contre le corps intermédiaire, procédé au cours duquel on rapporte sur le dispositif d'injection une tige de piston ou une seringue une fois que le dispositif de sécurité est en configuration de stockage. Au cours d'un tel assemblage, les moyens de désactivation ont une double fonction, d'une part éviter l'endommagement des moyens escamotables au cours de leur stockage, d'autre part éviter l'escamotage non désiré des moyens escamotables au cours de l'assemblage du dispositif d'injection, plus précisément au moment de l'assemblage du piston ou de la seringue sur le dispositif de sécurité.

L'invention sera mieux comprise à la lecture de la description qui va suivre, donnée uniquement à titre d'exemple et faite en se référant aux dessins dans lesquels :
- la figure 1 est une vue en coupe longitudinale schématique d'un dispositif de sécurité en configuration de stockage, selon un mode de réalisation;
- la figure 2 est une vue similaire à la figure 1, le dispositif de sécurité étant en configuration d'injection ;
- la figure 3 est une vue similaire à la figure 1, le dispositif de sécurité étant en configuration de sécurité ;
- la figure 4 est une vue explosée d'un dispositif d'injection d'implant selon un autre mode de réalisation ;
- la figure 5 est une vue en coupe longitudinale du dispositif de la figure 4, en configuration de stockage ;
- la figure 6 est une vue similaire à la figure 5, le dispositif d'injection étant en configuration d'injection ;
- la figure 7 est une vue similaire à la figure 5, le dispositif d'injection étant en configuration de sécurité ;
- la figure 8 est une vue en perspective d'un dispositif d'injection de liquide selon encore un autre mode de réalisation ; et
- la figure 9 est une vue en coupe longitudinale partielle du dispositif de la figure 8, en configuration de stockage.

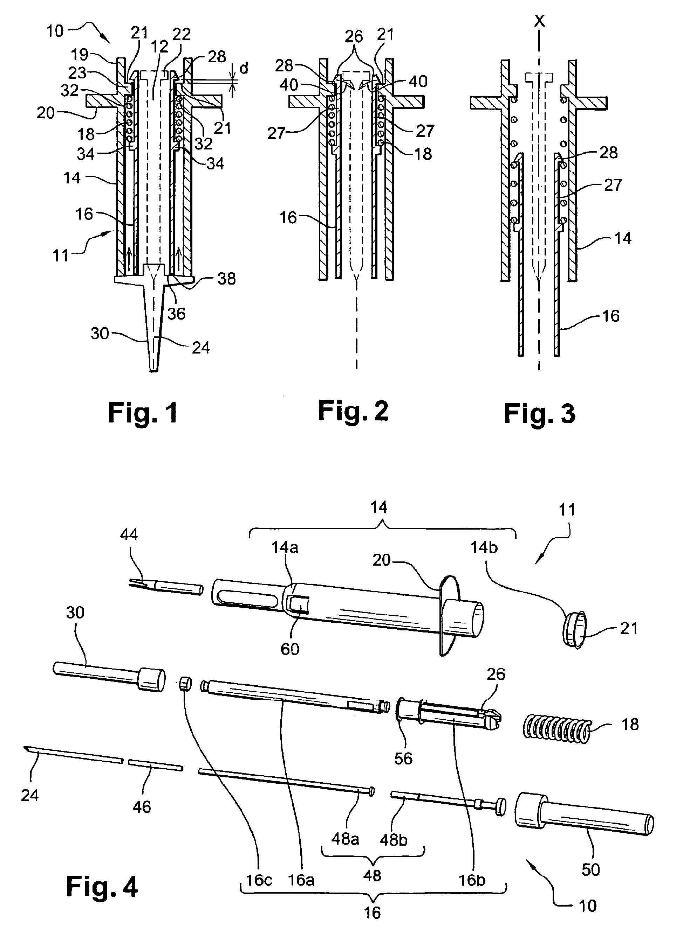

Un dispositif d'injection 10, comportant un dispositif de sécurité 11 selon un mode de réalisation et une seringue d'injection 12, est représenté sur les figures 1 à 3.

On notera que ces figures 1 à 3 sont très schématiques afin d'illustrer le fonctionnement proposé par l'invention, et ne représentent donc pas toutes les fonctionnalités du dispositif d'injection 10.

Comme cela est décrit dans la suite, le dispositif de sécurité 11 peut prendre au moins trois configurations distinctes, à savoir une configuration de stockage représentée sur la figure 1, une configuration d'injection représentée sur la figure 2 et une configuration de sécurité représentée sur la figure 3.

Le dispositif de sécurité comprend un corps intermédiaire 14 et un fourreau protecteur 16, monté coulissant à l'intérieur du corps intermédiaire 14, le fourreau 16 étant entraîné par des moyens de rappel 18. Les moyens de rappel son composés, ici, d'un ressort en compression 18.

Dans cet exemple, le corps intermédiaire 14, cylindrique, porte des moyens 20 de préhension du dispositif, en l'occurrence des ailettes 20, destinées à être saisies par l'utilisateur au cours de l'injection. Par ailleurs, le corps intermédiaire 14 comprend, sur son extrémité proximale 19, une surface de butée 21, portée dans cet exemple par deux saillies 23. Toujours dans cet exemple, le fourreau protecteur 16, cylindrique, est monté coulissant à l'intérieur du corps intermédiaire 14.

La seringue 12, représentée en pointillé, est munie d'un piston (non représenté). Dans cet exemple, la seringue 12 est en permanence solidarisée au corps intermédiaire 14, quelle que soit la configuration du dispositif de sécurité 11. Plus précisément, la seringue 12 comprend une collerette 22, encliquetée dans le corps intermédiaire 14, par des moyens non représentés. Par ailleurs, la seringue 12 porte, à son extrémité distale, une aiguille d'injection 24.

Le fourreau protecteur 16 peut prendre au moins trois positions distinctes dans le dispositif de sécurité 11, à savoir une position de stockage, une position d'injection et une position de sécurité, correspondant aux positions prises par le fourreau 16 dans les configurations respectives de stockage, d'injection et de sécurité du dispositif d'injection représenté sur les figures 1, 2 et 3. Dans la position d'injection représentée sur la figure 2, le fourreau protecteur 16 permet de laisser l'aiguille d'injection 24 découverte, de façon à la laisser pénétrer dans la peau d'un patient. Dans la position de sécurité illustrée sur la figure 3, le fourreau protecteur 16 permet de recouvrir l'aiguille d'injection 24, afin d'empêcher notamment des risques de contamination une fois l'injection faite.

Comme on peut le voir sur la figure 2, le fourreau protecteur 16 comporte des moyens 26 de retenue des moyens de rappel 18. Ces moyens de retenue 26 sont escamotables. Ils peuvent prendre une position désactivée, dans la configuration de stockage du dispositif, une position de retenue, dans la configuration d'injection, et une position escamotée, dans la configuration de sécurité du dispositif 11. Dans cet exemple, les moyens de retenue 26 comprennent deux pattes élastiques 27 diamétralement opposées, s'étendant dans la direction longitudinale X du dispositif, l'extrémité proximale des pattes 27, portant des ergots escamotables 28, étant à débattement élastique radial.

De même que le fourreau protecteur 16, les moyens de rappel 18 peuvent prendre des positions de stockage, d'injection et de sécurité, correspondant aux positions prises par les moyens de rappel 18 lorsque le dispositif 11 est en configuration respectivement de stockage, d'injection et de sécurité. A cet effet, comme moyens de retenue des moyens de rappel 18, outre les moyens escamotables 26, le dispositif 11 comprend une surface de butée proximale 32, portée dans cet exemple par les saillies 23 du corps intermédiaire 14, et une surface de butée distale 34, portée par deux saillies ménagées sur le fourreau protecteur 16. Ainsi, l'extrémité proximale des moyens de rappel 18 est en butée contre la surface 32, et leur extrémité distale est en butée contre la surface 34.

Le dispositif de sécurité 11 comporte en outre un capuchon de protection 30, recouvrant l'aiguille 24 lorsque le dispositif 11 est en configuration de stockage, monté par exemple par vissage sur le corps intermédiaire 14. Dans cet exemple, lorsque le capuchon 30 est vissé sur le dispositif 11, ce dernier prend sa configuration de stockage, comme cela est décrit dans la suite.

Le capuchon 30 compose ici des moyens de désactivation des moyens escamotables 26. Grâce à ces moyens de désactivation, le dispositif de sécurité 11 peut prendre la configuration de stockage de la figure 1, dans laquelle les moyens escamotables 26 ne sont pas soumis à la contrainte des moyens de rappel 18, comme cela découle du fonctionnement du dispositif dont la description est la suivante.

Dans la configuration de stockage illustrée sur la figure 1, le capuchon 30 est monté sur le corps intermédiaire 14 par vissage, ce vissage déclenchant un léger déplacement du fourreau protecteur 16, illustré par les flèches de la figure 1. Plus précisément, le capuchon 30 porte une surface de butée 36, se déplaçant dans la direction X lors du vissage du capuchon 30, et déplaçant ainsi l'extrémité distale 38 du fourreau protecteur 16. Du fait de ce déplacement du fourreau protecteur 16, les moyens escamotables 26 se déplacent également d'une distance d et ne sont plus en contact avec la surface de butée 21. Ainsi, les moyens de désactivation 30 maintiennent les ergots 28 à une distance d (illustrée sur la figure 1) de la butée 21 lorsque le dispositif est en configuration de stockage. Grâce à ce déplacement, les moyens de retenue 26 ne sont pas soumis à la contrainte des moyens de rappel 18, c'est-à-dire que ces moyens de rappel 18 n'exercent pas leur force de rappel sur les ergots 28. En fait, les moyens de rappel comprimés 18 exercent leur force de rappel sur les butées 32, 34, donc sur le capuchon 30 qui reçoit la poussée du fourreau protecteur 16 contre la butée 36, et non sur les moyens escamotables 26.

Dans cette configuration de stockage, comme expliqué plus haut, le dispositif de sécurité 11, avec ou sans la seringue 12, peut être stocké avant son utilisation, sans risquer de détériorer les moyens escamotables 26.

La configuration d'injection du dispositif 11 est représentée sur la figure 2. Dans cette configuration, l'aiguille 24 est laissée découverte, et peut donc être enfoncée dans la peau d'un patient afin d'injecter le produit. Pour passer de la configuration de stockage à la configuration d'injection, l'utilisateur ôte le capuchon 30, en le dévissant du corps intermédiaire 14. Comme le capuchon est retiré, le fourreau protecteur 16 n'est plus maintenu par la butée 36, si bien que les moyens de rappel 18, exerçant leur force sur la butée 34, déplacent le fourreau vers le bas sur une distance voisine de la distance d, jusqu'à ce que les ergots 28 rentrent en contact avec la butée 21. Comme on peut le voir sur la figure 2, les moyens escamotables 26 retiennent les moyens de rappel 18 dans la position d'injection, position dans laquelle le fourreau protecteur 16 laisse découverte l'aiguille et dans laquelle les moyens escamotables 26 sont en contact avec la butée 21. Dans cette configuration, le produit peut être injecté dans le corps du patient.

Une fois le produit injecté, le dispositif prend sa configuration de sécurité de la façon suivante. Lorsque le piston arrive en fin de course, les moyens escamotables 26 sont escamotés de la butée 21, par exemple en étant poussés vers l'intérieur par l'extrémité proximale de la tige de piston, conformément aux flèches 40 de la figure 2, si bien que les moyens 26, escamotés, n'exercent plus leur fonction de retenue des moyens de rappel 18. En conséquence, le fourreau protecteur 16 est projeté vers l'avant par les moyens de rappel 18 qui se détendent, recouvrant ainsi l'aiguille 24. Dans cette configuration, le dispositif de sécurité 11 protège l'aiguille, de façon à empêcher des contaminations après l'injection. On notera que la course du fourreau protecteur 16 est de préférence limitée par des butées prévues sur le corps intermédiaire 14 et le fourreau protecteur 16, non représentées.

Les figures 1 à 3 ont été représentées avec une seringue 12, permettant d'injecter un produit sous forme liquide, mais le dispositif de sécurité 11 peut être utilisé dans un dispositif d'injection d'un implant à l'état solide ou semi-solide, comprenant des moyens autres qu'une seringue d'injection de liquide.

Par ailleurs, on notera qu'un aspect particulièrement avantageux de l'assemblage du dispositif d'injection 10 réside dans le fait que l'on peut rapporter la seringue pré-remplie 12, munie de son piston, une fois que le dispositif de sécurité 11 est dans sa configuration de stockage. Ce mode de réalisation est intéressant du fait que l'on peut stocker le dispositif de sécurité 11 de façon indépendante de la seringue contenant le liquide. Grâce au dispositif décrit ci-dessus, lorsque l'on rapporte par la suite la seringue 12 munie de son piston, par exemple en encliquetant la collerette 22 dans le corps 14, on ne sollicite pas du tout les moyens escamotables 26, puisque ces moyens 26 ne sont pas soumis à la contrainte des moyens de rappel 18 dans la configuration de stockage. En d'autres termes, comme les moyens de rappel 18 n'exercent pas de force sur les moyens escamotables 26, mais sur le capuchon 30, l'insertion de la seringue 12, pouvant générer des mouvement brusques du dispositif 11, ne peut pas déclencher accidentellement l'escamotage des moyens 26, comme cela pourrait être le cas si l'on insérait la seringue 12 lorsque le dispositif de sécurité 11 est dans sa configuration d'injection, illustrée sur la figure 2.

On comprend qu'en configuration de stockage, les moyens de désactivation 30 exercent sur les moyens de rappel 18 une contrainte supérieure à la contrainte exercée par les moyens escamotables 26 en configuration d'injection.

On a représenté sur les figures 4 à 7 un exemple d'un dispositif d'injection d'un implant. Dans ce dispositif, les éléments ayant des fonctionnalités similaires à ceux du mode de réalisation des figures 1 à 3 sont désignés par des références identiques.

Le dispositif de sécurité 11 comprend un corps intermédiaire 14, un fourreau protecteur 16, des moyens de rappel 18, un capuchon de protection 30. Outre le dispositif de sécurité 11, le dispositif d'injection 10 comprend un organe 44 de réception d'un implant 46, une aiguille d'injection 24 et une tige de piston 48. La tige 48 comporte une partie distale 48a et une partie proximale 48b, séparée de la partie distale 48a. La partie distale n'est pas forcément séparée de la partie proximale. Enfin, le dispositif 10 comporte des moyens 50 d'immobilisation du piston 48, composés dans cet exemple d'un capuchon 50 destiné à recouvrir l'extrémité proximale du piston 48, et empêchant que le piston puisse être déplacé avant l'utilisation du dispositif.

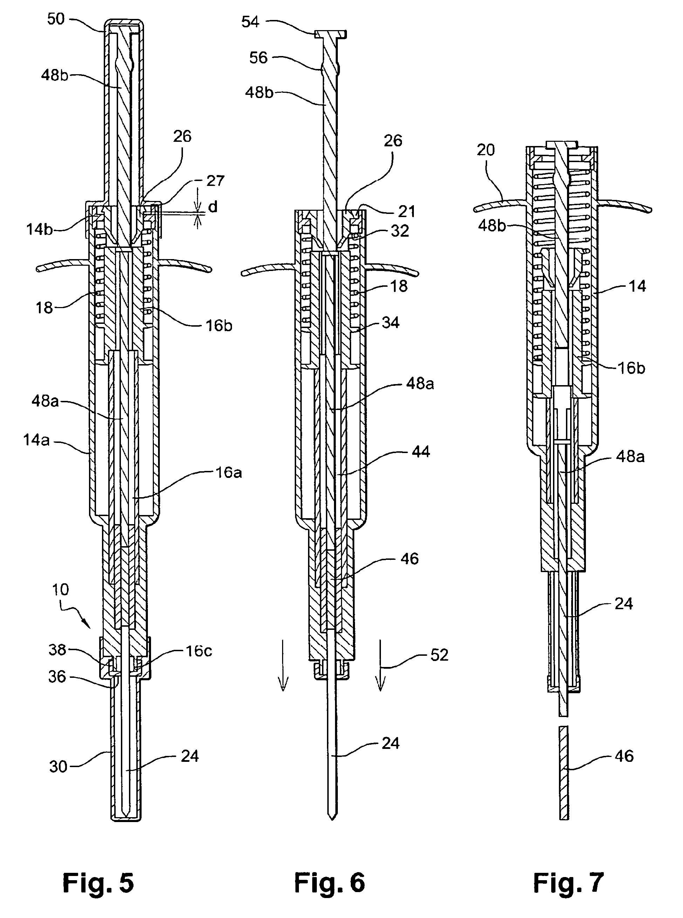

Dans cet exemple, le corps intermédiaire 14 est composé d'une partie principale 14a, portant les moyens de préhension 20, sur laquelle est rapporté un embout proximal 14b, portant notamment la surface de butée 21, visible sur les figures 5 à 7.

Toujours dans cet exemple, le fourreau protecteur 16 est muni d'une partie principale 16a et d'une extrémité proximale 16b, rapportée sur la partie 16a, portant notamment les moyens escamotables 26. De même que pour les figures 1 à 3, les moyens escamotables 26 comprennent deux ergots 28. Le fourreau protecteur 16 comporte en outre un organe distal 16c, rapporté sur la partie principale 16a.

Le fonctionnement du dispositif de la figure 4 va à présent être décrit, en référence aux figures 5 à 7.

Dans la configuration de stockage, illustrée sur la figure 5, le capuchon 30 de protection de l'aiguille 24 est vissé sur le corps intermédiaire 14, plus précisément sur l'extrémité distale de la partie 14a, ce qui a pour effet de déplacer le fourreau protecteur 16 par rapport au corps intermédiaire 14, si bien que les moyens escamotables 26 ne sont pas en contact avec la surface de butée 21, et ne sont donc pas soumis à la contrainte des moyens de rappel 18. En outre, dans cette configuration de stockage, le dispositif d'injection 10 est muni du capuchon 50 d'immobilisation du piston 48, empêchant le déplacement frauduleux ou accidentel du piston 48 lors du stockage du dispositif 10. Plus précisément, ce capuchon 50 est monté sur l'extrémité proximale du corps intermédiaire 14, par exemple par encliquetage. Comme le capuchon 50 est relativement rigide et comme il recouvre toute la partie du piston 48 qui dépasse de l'extrémité proximale du dispositif 10, il n'est pas possible que le piston 48 puisse être déplacé dans le dispositif 10.

Dans la configuration d'injection, illustrée sur la figure 6, le capuchon de protection de l'aiguille 30 et le capuchon d'immobilisation du piston 50 ont été retirés. Du fait du démontage du capuchon de protection 30, l'extrémité distale du fourreau 16 n'est plus en butée contre le capuchon 30. Le fourreau 16 est donc poussé par les moyens de rappel 18 dans la direction indiquée par les flèches 52, jusqu'à ce que les moyens escamotables 26 entrent en contact avec la surface 21 et exercent ainsi leur fonction de retenue des moyens de rappel 18, les retenant sous contrainte afin de maintenir le dispositif de sécurité en configuration d'injection. Dans cette configuration, on peut procéder à l'injection du produit, plus précisément de l'implant 46, à l'intérieur du corps du patient.

Dans ce mode de réalisation, le dispositif 10 est un dispositif de rétro-injection d'implant, permettant d'injecter l'implant 46 au cours du retrait de l'aiguille 24 hors du corps du patient. En effet, les moyens 24, 44, 48a, 48b sont des moyens de rétro-injection d'implant. Plus précisément, pour effectuer l'injection, on fait tout d'abord pénétrer l'aiguille à l'intérieur du corps du patient, puis on pousse sur le poussoir 54 de la partie 48b du piston 48, afin de faire sortir l'implant 46 l'organe de réception 44, et de l'introduire à l'intérieur de l'aiguille creuse 24. Une fois l'implant 46 à l'extrémité distale de l'aiguille creuse, le piston 48 est en fin de course, ce qui déclenche la rétro-injection automatique de l'implant 46. Cette rétro-injection comprend une étape d'escamotage des moyens 26 hors de la butée 21, cet escamotage étant exécuté par exemple par une saillie 56 de la partie 48b du piston. L'escamotage libère les moyens de rappel 18, qui se détendent et poussent ainsi le fourreau protecteur 16 vers le bas, grâce à la surface de butée 34. Sous l'effet de la poussée, la partie 16c entre en contact avec la peau du patient, si bien que le mouvement des moyens de rappel 18 pousse le corps intermédiaire 14 vers le haut, jusqu'à ce qu'il prenne la position finale de sécurité illustrée sur la figure 7. Au cours de cette remontée du corps intermédiaire 14, l'implant 46 est maintenu à la même distance par rapport à la peau du patient que lorsqu'il était à l'extrémité distale de l'aiguille 24. En effet, l'implant est maintenu à cette hauteur grâce à la partie 48a de la tige de piston, cette dernière ne remontant pas avec le corps intermédiaire 14, du fait qu'elle est désolidarisée de l'autre partie 48b solidaire du corps intermédiaire 14, comme on peut le voir sur la figure 7. Comme l'aiguille 24 est entraînée vers le haut avec le corps intermédiaire 14, le retrait de l'aiguille libère un certain espace dans la peau, espace qui est rempli par l'implant 46. A l'issue de cette rétro-injection, le dispositif 11 est en configuration de sécurité.

Dans la configuration de sécurité, visible sur la figure 7, le fourreau protecteur 16 recouvre complètement l'aiguille d'injection 24, et est retenu dans cette position grâce à des moyens d'encliquetage prévus sur le fourreau 16 et le corps intermédiaire 14, plus précisément grâce à une saillie 58 du fourreau protecteur 16, s'encliquetant dans des fentes 60 prévues sur le corps intermédiaire 14, visibles sur la figure 4.

L'invention n'est pas limitée aux modes de réalisation précédemment décrits. En particulier, les moyens de désactivation des moyens escamotables 26 prennent ici la forme d'un capuchon 30 de protection de l'aiguille. Néanmoins, ces moyens de désactivation pourraient prendre d'autres formes. Par exemple, on pourrait envisager que les moyens de désactivation soient prévus sur une pièce spécifique à la désactivation des moyens escamotables, ou encore sur l'une des autres pièces du dispositif d'injection 10, par exemple sur le capuchon 50 d'immobilisation du piston.

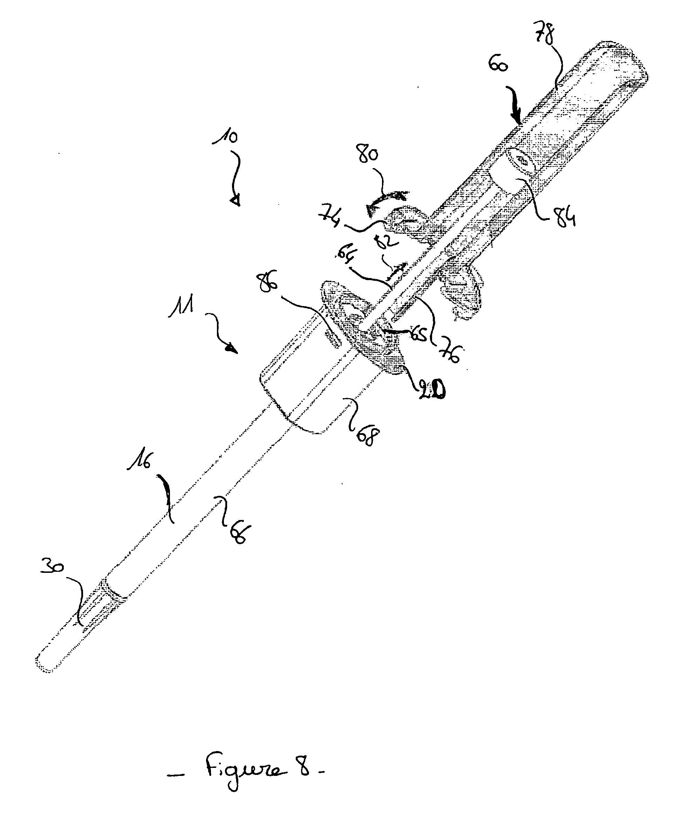

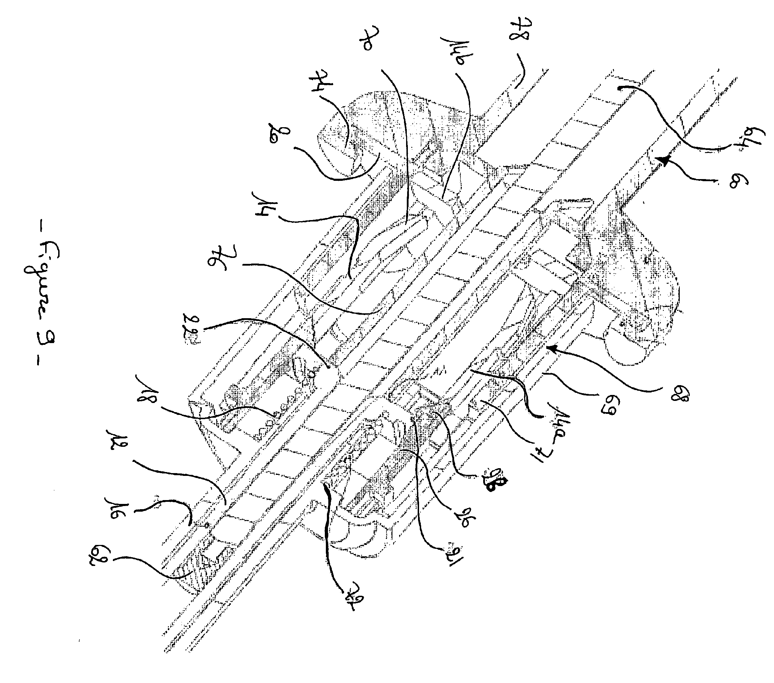

Un exemple de dispositif dans lequel les moyens de désactivation ne sont pas portés par le capuchon 30 est représenté sur les figures 8 et 9, décrites ci-après. Les éléments ayant des fonctionnalités similaires à ceux du mode de réalisation des autres figures ne sont pas décrits ou sont désignés par des références identiques.

Le dispositif d'injection 10 comprend le dispositif de sécurité 11 et la seringue 12 d'injection de liquide ou de gel, la seringue étant munie d'un capuchon 30 rapporté à son extrémité distale, d'un piston 62 et d'une tige de piston 64 à son extrémité proximale. Le dispositif de sécurité 11 comprend un fourreau protecteur 16, un corps intermédiaire 14, des moyens de rappel 18 et un organe de stockage 60.

Le fourreau 16 comprend dans cet exemple une partie distale 66 tubulaire et une partie proximale 68. Cette partie proximale 68 du fourreau comprend deux pièces rapportées et fixées l'une à l'autre de façon permanente : d'une part une enveloppe extérieure 69, prolongeant le tube de la partie distale 66, d'autre part une pièce intérieure 71, encliquetée dans l'enveloppe 69. La pièce intérieure 71 porte d'une part, à son extrémité proximale, des ailettes de préhension 20, et d'autre part des moyens escamotables 26 de retenue des moyens de rappel 18, comprenant ici deux pattes élastiques diamétralement opposées, portant des ergotes escamotables 28. La partie proximale 68 reçoit le corps intermédiaire 14 lorsque le dispositif 10 est en configuration de stockage ou d'injection. Dans cet exemple, les ailettes 20 forment des moyens de fixation de l'organe de stockage 60 sur le dispositif de sécurité 11, et des rampes 65 sont prévues pour dégager l'organe 60 du dispositif, comme décrit dans la suite.

Le corps intermédiaire 14 a sensiblement la forme d'un capuchon, il comprend des parois latérales 14a et une paroi transversale supérieure 14b, traversée par la tige de piston 64. Le corps 14 comporte des moyens 70 d'encliquetage de la seringue 12 en configuration de sécurité, comme décrit dans la suite.

Les moyens de rappel 18 comprennent un ressort en compression, en appui d'une part contre un siège 72 du fourreau 16, d'autre part contre la collerette 22 de la seringue 12.

L'organe de stockage 60 est rapporté sur l'extrémité proximale du dispositif. Il comprend des moyens amovibles 74 de fixation de l'organe 60 sur le dispositif 11, une partie distale 76 et une partie proximale 78. La partie distale 76 est semi-tubulaire. Elle s'étend depuis les moyens de fixation 74 vers les moyens de rappel 18 et forme entretoise entre les moyens de fixation 74 et les moyens de rappel 18. Plus précisément, la partie distale 76, en configuration de stockage, appuie indirectement sur les moyens de rappel 18, en appuyant sur la collerette 22 sur laquelle agissent les moyens 18, de façon à maintenir les ergots 28 à une certaine distance d de la butée 21 ménagée sur la collerette 22. Les moyens de fixation 74 permettent de fixer, de façon amovible, l'organe 60 sur la partie 68 du fourreau 16. Ils comprennent, dans cet exemple, des fentes pouvant enserrer les ailettes 20 de la partie 68. L'organe de stockage 60 comprend en outre des moyens de protection de la tige de piston 64, portés par la partie proximale 78. Plus précisément, cette partie proximale 78 a une forme semi-tubulaire ouverte, de façon à recevoir toute la tige de piston 64 et former ainsi un étui de protection, empêchant la déformation de la tige et limitant son accès.

On comprend que l'organe de stockage 60 forme des moyens de désactivation des moyens escamotables 26 au cours du stockage, en exerçant sur les moyens de rappel 18 une contrainte supérieure à la contrainte exercée par les moyens escamotables 26 en configuration d'injection.

Le fonctionnement du dispositif des figures 8 et 9 va à présent être décrit.

Lorsque le dispositif est en configuration de stockage, l'organe 60 est fixé sur le dispositif de sécurité 11, par coopération des fentes 74 et des ailettes 20. Dans cette configuration, la partie 76 appuie sur le ressort 18 de façon à exercer une contrainte supérieure à celle exercée par les moyens 26 en configuration d'injection. Plus précisément, l'organe 60 appuie sur la seringue 12 de manière à ne pas solliciter les ergots escamotables 28. Tant que l'organe 60 est en place, les moyens 26 ne subissent aucun effort de la part du ressort 18. On notera que, grâce à la partie 78, l'organe 60 assure une protection de la tige de piston 64, notamment en limitant son accès.

Afin de procéder à l'injection, on retire l'organe de stockage 60, en le faisant pivoter, conformément à la flèche 80, de façon à dégager les fentes 74 des ailettes 20. Par coopération entre l'organe 60 et les rampes 65, l'organe 60 est déplacé conformément à la flèche 82, ce qui a pour effet de le dégager complètement du dispositif 11. Une fois l'organe 60 démonté du dispositif 11, le dispositif prend automatiquement sa configuration d'injection. En effet, sous l'effet du ressort 18, la collerette 22 de la seringue est plaquée contre les ergots 28, qui assurent sa retenue. On peut ensuite retirer le capuchon 30, de façon à découvrir l'aiguille 24 et à l'introduire dans la peau du sujet. On injecte le produit par pression sur l'extrémité proximale 84 de la tige de piston 64.

Une fois la dose de produit intégralement injectée, le piston arrive en fin de course, si bien que l'extrémité proximale 84 de la tige arrive en contact avec la partie 14b du corps intermédiaire 14, ce qui le fait coulisser dans le fourreau jusqu'à ce que les parois 14a entrent en contact avec les ergots escamotable 28. En appuyant encore sur l'extrémité 84, les ergots 28 sont écartés vers l'extérieur, par effet de rampe, si bien qu'ils n'exercent plus de retenue sur la seringue 12. Aussi, en relâchant la pression sur l'extrémité 84, l'utilisateur fait remonter la seringue 12 relativement au fourreau 16, sous l'action du ressort 18 qui se détend. La remontée de la seringue a pour effet, d'une part, d'encliqueter la collerette 22 dans les moyens d'encliquetage 70, d'autre part de faire coulisser le corps intermédiaire dans la direction 82 de façon à le faire sortir de la partie 68 du dispositif. En fin de course, le corps intermédiaire 14 est immobilisé par rapport au fourreau 16, par encliquetage d'ergots ménagés sur le corps intermédiaire 14, dans des fentes 86 ménagées sur la partie proximale 68 du fourreau 16. Dans cette position, le dispositif 11 est en configuration de sécurité, l'aiguille 24 est recouverte par le fourreau 16, évitant toute piqûre accidentelle.

On comprendra que grâce aux dispositifs de sécurité décrits ci-dessus, on épargne les moyens escamotables 26 en configuration de stockage, ce qui garantit que ces moyens escamotables 26 ne sont pas usés au moment de l'utilisation du dispositif de sécurité 11. Grâce à cette protection des moyens escamotables 26, on peut prévoir de réaliser ces moyens 26 différemment, par exemple dans un matériau plus souple.

On comprendra que les dispositifs de sécurité proposés ci-dessus peuvent être utilisés aussi bien dans un dispositif d'injection comprenant une seringue d'injection de liquide ou de gel, que dans un dispositif d'injection permettant d'introduire un implant sous forme solide ou semi-liquide.

On notera que l'invention peut prendre d'autres formes que celles décrites précédemment. Par exemple, le piston 48 des figures 5, 6, 7 est réalisé en deux parties dans l'exemple, mais pourrait également être réalisé sous forme d'une tige monobloc, ce qui est avantageux, notamment car cela diminue le nombre et la forme des pièces à assembler.

## Revendications

1. Dispositif de sécurité (11) pour dispositif d'injection (10) comportant : ,
- un fourreau protecteur (16) entraîné par des moyens de rappel (18), le dispositif de sécurité (11) pouvant prendre une configuration d'injection, dans laquelle le fourreau (16) permet de laisser une aiguille d'injection (24) découverte, et une configuration de sécurité, dans laquelle le fourreau (16) permet de recouvrir l'aiguille d'injection (24),
- des moyens escamotables (26) de retenue des moyens de rappel (18), retenant les moyens de rappel (18) sous contrainte de façon à maintenir le dispositif de sécurité (11) en configuration d'injection,
- des moyens (30, 60) de désactivation des moyens escamotables (26), permettant au dispositif de sécurité (11) de prendre une configuration de stockage dans laquelle les moyens escamotables (26) ne sont pas soumis à la contrainte des moyens de rappel (18), dispositif **caractérisé en ce que** les moyens (30) de désactivation des moyens escamotables (26), dans la configuration de stockage, exerçent sur les moyens de rappel (18) une contrainte supérieure à la contrainte exercée par les moyens escamotables (26) en configuration d'injection.

2. Dispositif selon la revendication précédente, dans lequel les moyens escamotables (26) de retenue des moyens de rappel (18) comprennent des ergots escamotables (28), ces ergots étant en butée contre une surface (21) lorsque le dispositif (11) est en configuration d'injection, et escamotés de cette butée (21) lorsque le dispositif est en configuration de sécurité ; les moyens de désactivation (30, 60) maintenant les ergots (28) à une certaine distance (d) de la butée (21) lorsque le dispositif est en configuration de stockage.

3. Dispositif selon l'une quelconque des revendications précédentes, dans lequel les moyens de désactivation comprennent un capuchon de protection (30) recouvrant l'aiguille (24) lorsque le dispositif (11) est en configuration de stockage, le montage du capuchon (30) sur le dispositif de sécurité (11) déclenchant la désactivation des moyens escamotables (26), de préférence en déplaçant le fourreau protecteur (16).

4. Dispositif selon l'une quelconque des revendications 1 à 3, dans lequel les moyens de désactivation comprennent un organe de stockage (60) monté sur l'extrémité proximale du dispositif (11) et appuyant, directement ou indirectement, sur les moyens de rappel (18) pour leur faire prendre leur configuration de stockage.

5. Dispositif selon la revendication précédente, l'organe (60) comprenant des moyens amovibles (74) de fixation sur le dispositif (11) et une partie distale (76), s'étendant depuis les moyens de fixation (74) et formant entretoise entre les moyens de fixation (74) et les moyens de rappel (18).

6. Dispositif selon la revendication 4 ou 5, dans lequel l'organe de stockage (60) comprend des moyens (78) de protection d'une tige de piston (64).

7. Dispositif d'injection (10) comportant un dispositif de sécurité (11) selon l'une quelconque des revendications précédentes.

8. Dispositif selon la revendication précédente, le dispositif étant un dispositif d'injection d'un implant, et comportant des moyens (24, 44, 48a, 48b) de rétro-injection de l'implant.

9. Dispositif selon la revendication 7, le dispositif étant un dispositif d'injection d'un liquide ou de gel, et comportant une seringue (12) d'injection de liquide.

10. Procédé d'assemblage d'un dispositif d'injection (10) comportant un dispositif de sécurité selon l'une quelconque des revendications 1 à 6, le fourreau (16) étant monté coulissant par rapport à un corps intermédiaire (14), et les moyens de rappel (18) étant retenus, d'une part contre le fourreau (16), d'autre part contre le corps intermédiaire (14), procédé au cours duquel on rapporte une tige de piston (48a, 48b) ou une seringue (12) sur le dispositif d'injection (10) une fois que le dispositif de sécurité (11) est en configuration de stockage.

## Claims

1. Safety device (11) for an injection device (10), comprising:
• a protective sheath (16) driven by return means (18), the safety device (11) being capable of taking an injection configuration in which the sheath (16) allows an injection needle (24) to be left uncovered, and a safety configuration in which the sheath (16) enables the injection needle (24) to be covered;
• retractable means (26) for retaining the return means (18), the retractable means retaining the return means (18) under stress in such a manner as to hold the safety device (11) in the injection configuration; and
• deactivation means (30, 60) for deactivating the retractable means (26), enabling the safety device (11) to take a storage configuration in which the retractable means (26) are not subjected to stress from the return means (18), the device (11) being **characterized in that** the means (30) for deactivating the retractable means (26) in the storage configuration exert stress on the return means (18) that is greater than the stress exerted thereon by the retractable means (26) when in the injection configuration.

2. Device according to the preceding claim, wherein the retractable means (26) for retaining the return means (18) comprise retractable catches (28), the catches being in abutment against a surface (21) when the device (11) is in the injection configuration and being retracted from said abutment (21) when the device is in the safety configuration; the deactivation means (30, 60) holding the catches (28) at a certain distance (d) from the abutment (21) when the device is in the storage configuration.

3. Device according to any preceding claim, wherein the deactivation means comprise a protective cap (30) covering the needle (24) when the device (11) is in the storage configuration, fitting the cap (30) on the safety device (11) causing the retractable means (26) to be deactivated, preferably by moving the protective sheath (16).

4. Device according to any one of claims 1 to 3, wherein the deactivation means comprise a storage member (60) mounted on the proximal end of the device (11) and bearing directly or indirectly against the return means (18) to cause them to take up their storage configuration.

5. Device according to the preceding claim, the member (60) comprising removable fastener means (74) on the device (11) and a distal portion (76) extending from the fastener means (74) and forming a spacer between the fastener means (74) and the return means (18).

6. Device according to claim 4 or claim 5, wherein the storage member (60) includes means (78) for protecting a piston rod (64).

7. Injection device (10) including a safety device (11) according to any preceding claim.

8. Device according to the preceding claim, the device being a device for injecting an implant and including implant retro-injection means (24, 44, 48a, 48b).

9. Device according to claim 7, the device being a device for injecting a liquid or a gel and comprising a liquid injection syringe (12).

10. Method of assembling an injection device (10) including a safety device according to any one of claims 1 to 6, the sheath (16) being slidably mounted relative to an intermediate body (14), and the return means (18) being retained firstly against the sheath (16) and secondly against the intermediate body (14), in which method a piston rod (48a, 48b) or a syringe (12) is fitted to the injection device (10) once the safety device (11) is in the storage configuration.

## Patentansprüche

1. Sicherheitsvorrichtung (11) für eine Injektionsvorrichtung (10), die umfasst:
- eine Schutzhülle (16), die durch eine Rückholeinrichtung (18) angetrieben wird, wobei die Sicherheitsvorrichtung (11) eine Injektionskonfiguration, in welcher die Hülle (16) eine Injektionsnadel (24) offen lässt, und eine Sicherungskonfiguration, in welcher die Hülle (16) die Injektionsnadel (24) bedeckt, einnehmen kann,
- Einrasteinrichtungen (26) zum Halten der Rückholeinrichtungen (18), die die Rückholeinrichtungen (18) unter Spannung halten, so dass die Sicherheitsvorrichtung (11) in der Injektionskonfiguration gehalten wird,
- Einrichtungen (30, 60) zum Deaktivieren der Einrasteinrichtungen (26), so dass die Sicherheitsvorrichtung (11) eine Lagerungskonfiguration einnehmen kann, in welcher die Einrasteinrichtungen (26) nicht durch die Rückholeinrichtungen (18) vorgespannt werden,
**dadurch gekennzeichnet, dass**
die Einrichtungen (30) zum Deaktivieren der Einrasteinrichtungen (26) in der Lagerungskonfiguration auf die Rückholeinrichtungen (18) eine Vorspannung ausüben, die größer als die Vorspannung ist, die durch die Einrasteinrichtungen (26) in der Injektionskonfiguration ausgeübt wird.

2. Vorrichtung nach dem vorangehenden Anspruch, bei der die Einrasteinrichtungen (26) zum Halten der Rückholeinrichtungen (18) Einrastnocken (28) umfassen, wobei die Nocken im Anschlag stehen gegen eine Oberfläche (21), wenn sich die Vorrichtung (11) in der Injektionskonfiguration befindet, und in Bezug auf diesen Anschlag (21) eingezogen sind, wenn sich die Vorrichtung in der Sicherungskonfiguration befindet; wobei die Einrichtungen zum Deaktivieren (30, 60) die Nocken (28) in einem vorgegebenen Abstand (d) von dem Anschlag (21) halten, wenn sich die Vorrichtung in der Lagerungskonfiguration befindet.

3. Vorrichtung nach einem der vorangehenden Ansprüche, bei welcher die Einrichtungen zum Deaktivieren eine Schutzkappe (30) umfassen, die die Nadel (24) bedeckt, wenn sich die Vorrichtung (11) in der Lagerungskonfiguration befindet, wobei das Aufsetzen der Halterung der Kappe (30) auf der Sicherheitsvorrichtung (11) das Deaktivieren der Einrasteinrichtungen (26) auslöst, vorzugsweise zugleich mit dem Verschieben der Schutzhülle (16).

4. Vorrichtung nach einem der Ansprüche 1 bis 3, bei der die Einrichtungen zum Deaktivieren ein Lagerungsorgan (60) umfassen, das an dem abseitigen Ende der Vorrichtung (11) angeordnet ist und sich direkt oder indirekt auf die Rückholeinrichtungen (18) stützt, so dass diese die Lagerungskonfiguration einnehmen können.

5. Vorrichtung nach einem der vorangehenden Ansprüche, bei der das Organ (60) Aufsatzeinrichtungen (74) zum Befestigen der Vorrichtung (11) umfasst und sich ein diesseitiger Abschnitt (76) von den Befestigungseinrichtungen (74) aus erstreckt und einen Steg zwischen den Befestigungseinrichtungen (74) und den Rückholeinrichtungen (18) bildet.

6. Vorrichtung nach Anspruch 4 oder 5, bei der das Lagerungsorgan (60) Einrichtungen (78) zum Schutz einer Kolbenstange (64) umfasst.

7. Injektionsvorrichtung (10), die eine Sicherheitsvorrichtung (11) nach einem der vorangehenden Ansprüche umfasst.

8. Vorrichtung nach dem vorangehenden Anspruch, wobei die Vorrichtung eine Injektionsvorrichtung für ein Implantat ist und Einrichtungen (24, 44, 48a, 48b) zur Retro-Injektion eines Implantats ist.

9. Vorrichtung nach Anspruch 7, bei der die Vorrichtung eine Injektionsvorrichtung für eine Flüssigkeit oder ein Gel ist und eine Spritze (12) zum Injizieren von Flüssigkeiten umfasst.

10. Verfahren zum Herstellen einer Injektionsvorrichtung (10), die eine Sicherheitsvorrichtung nach einem der Ansprüche 1 bis 6 umfasst, wobei die Hülle (16) gleitend in Bezug auf einen Zwischenkörper (14) montiert ist und die Rückholeinrichtungen (18) einerseits gegen die Hülle (16) und andererseits gegen den Zwischenkörper (14) gehalten werden, wobei man im Verlauf des Verfahrens eine Kolbenstange (48a, 48b) oder eine Spritze (12) auf die Injektionsvorrichtung (10) aufsteckt, sobald sich die Sicherheitsvorrichtung (11) in Lagerungskonfiguration befindet.
